# EUROPEAN PATENT APPLICATION

(11) **EP 2 620 152 A1**
(43) Date of publication of application: **31.07.2013**
(21) Application number: 11825509.0
(22) Date of filing: 11.05.2011
(51) Int. Cl.: A61K 31/545, A61K 9/51, A61K 47/04, A61P 31/04

(54) **PHARMACEUTICAL COMPOSITION WITH ANTIMICROBIAL ACTIVITY FOR PARENTERAL ADMINISTRATION AND PROCESS FOR PREPARING SAME**

(30) Priority: 13.09.2010 RU 2010001449
(71) Applicant: Limonov, Viktor Lvovich, Moscow 121374 (RU)
(72) Inventor: GAIDUL, Konstantin Valentinovich, Novosibirsk 630089 (RU); DUSHKIN, Aleksandr Valerevich, Novosibirsk 630117 (RU)
(74) Representative: von Füner, Nicolai
(86) International application number: PCT/RU2011/000320
(87) International publication number: WO 2012/036585

(57) **Abstract**

This invention is related to pharmacology, medicine, veterinary science and pharmaceutical industry. It particularly concerns a process for producing original composite antimicrobial preparations for parenteral administration, which possess a higher therapeutic efficiency in case of severe infections and inflammatory diseases.

The proposed pharmaceutical compositions contain as active agents betalactam antibiotics and finely dispersed nanostructured silica dioxide in a weight ratio of from 10:1 to 75:1 respectively.

The silica dioxide particles present in the composition are agents for delivering antibiotic molecules into the phagocytes which allows to increase the concentration of antimicrobial preparations at inflammation areas and considerably decrease the antibiotic resistance of microorganisms.

The proposed process for preparing the pharmaceutical composition includes mixing betalactam antibiotic with finely dispersed nanostructured silica dioxide and is characterized in that the mixture of the above-mentioned substances in a weight ratio of from 10:1 to 75:1 respectively is exposed to mechanical treatment by impact and abrasive actions until the fine powder fraction weight proportion increases (< 5 micron) until it becomes not less than 25%.

The obtained mixture is used for preparing injectable solutions.

## Description

This invention belongs to antimicrobial pharmaceutical preparations and their production technologies. It can be used in medicine and veterinary science for treating contagious and inflammatory diseases, as well as in the pharmaceutical industry for preparing medicinal products.

Currently, the most successful therapy for curing contagious and inflammatory diseases is based on using different anti-infectives, including betalactam antibiotics.

Betalactams are preparations (natural and semisynthetic penicillins, cephalosporins, cephamycins, carbapenems and monobactams) with a betalactam ring as a common fragment in the chemical structure, which determines the antimicrobial activity and a series of common properties of this group of drug preparations [1].

All betalactams possess a wide antimicrobial spectrum and a high level of antimicrobial activity, but many of them have a fast developing microbial resistance because of their production of specific ferments - betalactamase (extended spectrum betalactamase, chromosomal betalactamase class C, etc.), which hydrolyze the betalactam ring. This is what deprives these preparations of their antibacterial properties and leads to the development of resistant strains of microorganisms [2].

In the past decades there have been created specific betalactamase inhibitors (clavulanic acid, sulbactam, tazobactam and etc.), and on their basis there has been developed an entire range of effective combined antibacterial betalactam preparations of the penicillin and cephalosporin family (amoxicillin/clavulanic acid, ampicillin/sulbactam, piperacillin/tazobactam, cefoperazone/sulbactam and etc.) which are characterized by having an increased persistence to betalactamase as well as an increased apparent antibacterial activity [2, 3].

Nevertheless it ought to be remarked that many of these "inhibitor screened" preparations appeared to be insufficiently effective because in case of high betalactamase production by germs the inhibitors cannot fully protect the antibiotics from hydrolysis.

The carbapenems which are resistant to the action of many betalactamases cannot entirely solve the problem of microbial resistance to the mentioned antibiotics. It happens because many application ways for treating serious infections lead to the development of the multiply resistant strains *P. Aeruginosa* [3]*.*

Besides, frequently the clinical betalactam ineffectiveness (or their low effectiveness) in case of infections induced by different microbes is associated not only with the negative betalactamase activity, but also with the fact that these preparations have limited ability of local concentration at contagious inflammation locus and macrophage penetration, where many contagious and inflammatory diseases' activators are deposited. The antimicrobial resistance level depends on their functional status intensity [4, 5].

In the last few years it has been discovered that the use of different nanoparticles as dosing vehicle for the delivery of different antibiotics (such as e.g. betalactams) inside the bacteria and macrophages to increase their concentration at the contagious inflammation area and to increase their antimicrobial properties as well as the stimulation of the functional activity of the phagocytes (neutrophils and macrophages) and their additional recruitment to infected tissues is a very challenging trend for modern experimental pharmacology and clinical medicine [6, 7, 8, 9, 10, 11, 12].

The essence of the present invention is to increase the therapeutic effectiveness of betalactams by using SiO₂ (silica dioxide) nanoparticles, which, due to the fact that they are characterized by having pharmacologically beneficial biocompatibility, biodistribution, biodegradation and low toxicity properties (independent from looseness of the structure intensity), can serve as antibiotics carrier for endocellular macrophage delivery, which are concentrated at the inflammatory tissues of lungs, liver, kidneys, spleen, lymph glands, heart, skin, bladder and other mammalian organs (i.e. a considerably increase of the antibiotics concentration in the infected areas), and also initiate the antimicrobial activity of the immune system cells. This will help to authentically increase the germicidal therapeutic effect during the treatment of contagious inflammatory diseases [13, 14, 15, 15, 17, 18, 19, 20, 21].

The mentioned invention solves the issue of providing an injectable pharmaceutical composition having an antimicrobial action on the basis of using antibiotics from the group of betalactams and silica dioxide nanoparticles which possess a higher therapeutic effectiveness (compared to standard betalactams which are considered as basis for this invention) for the treatment of contagious and inflammatory diseases.

To solve the assigned task it is suggested to use an injectable pharmaceutical composition having an antimicrobial action, which contains a betalactam antibiotic and finely dispersed nanostructured silica dioxide in a weight ratio of (10-75) : 1.

For solving the assigned task it is suggested to prepare the injectable pharmaceutical composition having an antimicrobial action by mixing the betalactam antibiotics with other components. The betalactam antibiotic powder is mixed with the finely dispersed nanostructured silica dioxide powder in a weight ratio of (10-75) : 1, and the obtained mixture is subjected to mechanical treatment by impact abrasive actions.

The therapeutic effectiveness of the proposed pharmaceutical composition will increase if the obtained mixture is subjected to mechanical treatment in the form of impact abrasive actions in a way that the part of finely dispersed nanostructured silica dioxide particles with a size of not larger than 5 microns would be not less than 25%.

To prepare the mentioned pharmaceutical composition there were used foreign betalactam antibiotics provided by the Russian pharmacological company LLC "ABOLmed" (penicillins: carbenicillin; cephalosporins: cefazolin, cefuroxime, cefotaxime, ceftriaxone, cefoperazone, ceftazidime, cefoperazone/sulbactam, cefepime; cephamycims: cefoxin; carbapenems: meropenem; monobactams: aztreonam). As a finely dispersed nanostructured silica dioxide (hereafter referred to as BHSiO₂) was used "Palysorb" drug (pharmacological group: enterosorbing solution; active substance: colloidal silica dioxide), produced by the Russian company CJSC "Polysorb", containing round shaped silica dioxide nanoparticles (dimension 5-20 nm) combined into aggregates (irregular microparticles) with a dimension of ≤ 90 micron (registration number Nº 001140101-100908). There is s similar preparation produced by the Ukrainian company CJSC "Biopharma" with a trade name "Silics" [12].

The make-up of the composition is based on the phenomenon of convertible betalactam molecule sorption by nano- and micro BHSiO₂ particles, as well as BHSiO₂ particle reduction when mixtures thereof are mechanically activated with substances comprising betalactams by intensive mechanical impact abrasive treatment.

The process according to the present invention for preparing the previously mentioned pharmaceutical composition by the mechanical activation of the powdery mixture of betalactam antibiotic and BHSiO₂ with intensive impact abrasive operations allows to increase the amount of the finely divided BHSiO₂ particles (less than 5 micron) on which betalactam molecules are adsorbed and which are mostly phagocyted by macrophages [10,19].

To achieve this goal, the mixture of the above-mentioned materials in a weight ratio of betalactam antibiotic : BHSiO₂ equal (10-75) : 1 is exposed to mechanical activation by way of intensive impact abrasive actions until the weight portion of the finely divided fraction is increased up to 25%.

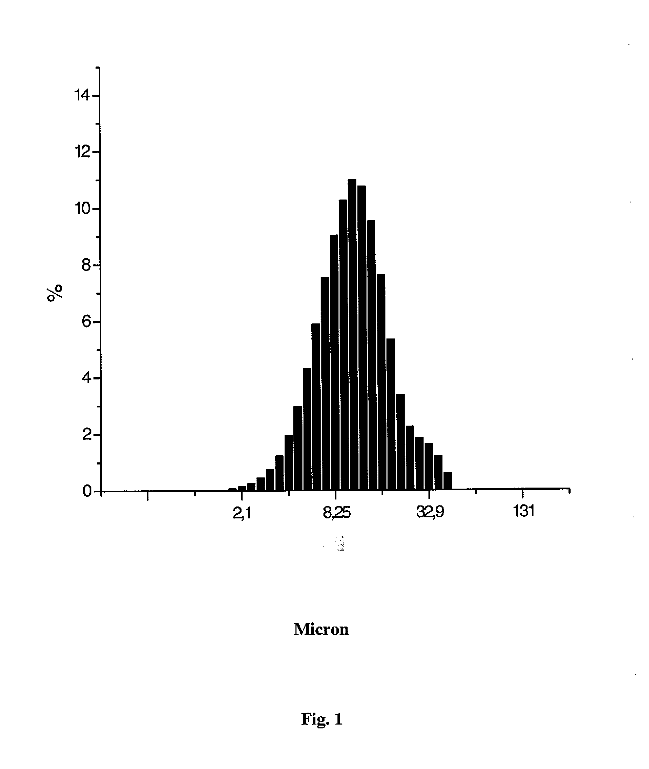

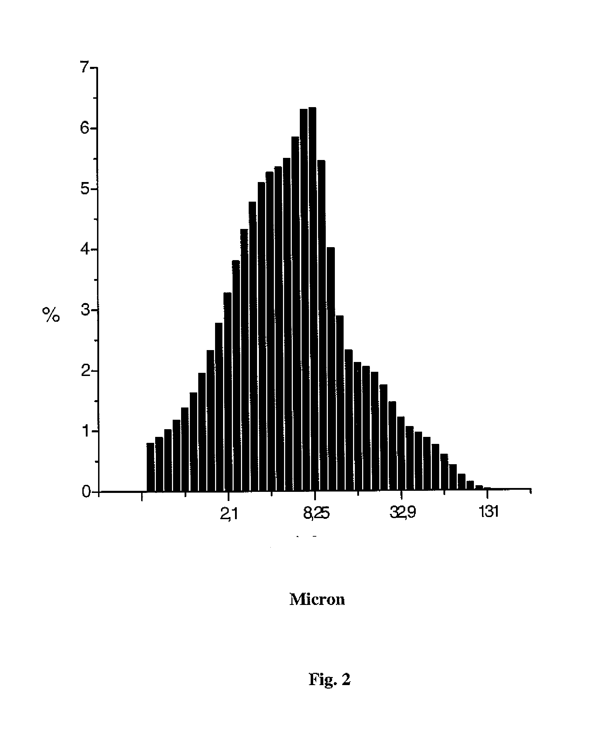

The data of the fractional make-up of the aqueous slurry in terms of ceftriaxone: BHSiO₂ equal 30 : 1, by weight, measured by a laser granulometer Micro-Sizer 201, are shown in Figs. 1 and 2.

As it is shown in Figs. 1 and 2, the analyzed composition having undergone a mechanical activation for a period of two hours leads to an increase of the weight rate of its finely dispersed fraction (particles dimension < 5 micron) which contains not less than 25%.

Injectable colliodal solutions for parenteral insertion can be prepared from the obtained powdery composition (by dissolving it using any known method appropriate for betalactams). Said solutions consist of finely dispersed BHSiO₂ particles with molecules of some betalactams inversibly sorbed on the surface thereof.

Table No. 1 contains data (received by high performance liquid chromatography method - HPLC) about the sorption rate of different betalactam antibiotics on BHSiO₂, particles after mechanical activation of antibiotics composition : BHSiO₂, in a weight ratio of 30 : 1, showing that the finely dispersed nanostructured silica dioxide can be used for parenteral administration as a dosing vehicle for antibiotics and other pharmacons which are capable of sorbing on the nano- and microparticles of this inorganic matter to be delivered to the inflammation areas, tumor growth areas, regeneration areas, cicatrization areas, scarring areas etc., i.e. into the areas with increased macrophages presence to purposefully increase the local (such as the intracellular) concentration of the pharmaceutical preparation and its therapeutic effect.

**Table No. 1**

| **Betalactam sorption rate by BHSiO₂* particles** | |
|---|---|
| Composition formulation, m/a time** | Quantity of sorbed antibiotic : quantity of BHSiO₂, mg (weight %) |
| Cefazolin:BHSiO₂ (30:1), m/a 2 hours | 8,1 mg : 16,7 mg (48%) |
| Ceftriaxone:BHSiO₂ (30:1), m/a 2 hours | 14,5 mg: 16,7 mg (85%) |
| Cefotaxime:BHSiO₂ (30:1), m/a 2 hours | 9,4 mg : 16,7 mg (55%) |
| Cefuroxime:BHSiO₂ (30:1), m/a 2 hours | 7,4 mg : 16,7 mg (44%) |
| Cefepime:BHSiO₂ (30:1), m/a 2 | 16,1 mg : 16,7 mg (96%) |
| Cefoperazone:BHSiO₂ (30:1), m/a 2 hours | 12,2 mg :16,7 mg (73%) |
| Cefoperazone/sulbactam: BHSiO₂ (30:1), m/a 2 hours | 13,9 mg : 16,7 mg (83%) |
| Ceftazidime:BHSiO₂ (30:1), m/a 2 hours | 9,6 mg : 16,7 mg (53%) |
| Cefoxotin:BHSiO₂ (30:1), m/a 2 hours | 8,5 mg : 16,7 mg (51%) |
| Meropenem:BHSiO₂ (30:1), m/a 2 hours | 10,6 mg : 16,7 mg (63%) |
| Aztreonam:BHSiO₂ (30:1), m/a 2 hours | 9, 7 mg: 16,7 mg (58%) |
| Carbenicillin:BHSiO₂ (30:1), m/a 2 hours | 11,2 mg : 16,7 mg (67%) |

| | |
|---|---|
| * - finely dispersed nanostructured silica dioxide **- mechanical activation | |

The introducing of the finely dispersed nanostructured silica dioxide in the ratio of betalactam : BHSiO₂ from 10:1 to 75:1 regarding its weight is determined by the combination of 2 factors: 1) during an increase of the BHSiO₂ content above 10% of the composition weight in case of laboratory animals, they suffer from the small capillary tube blockage in parenchymatous viscus; 2) in case of decreasing the BHSiO₂ content below 1% of the composition weight (in particular during mice treatment of bacterial sepsis) its therapeutic efficiency doesn't differ from the initial antibiotic basic efficiency.

For obtaining the composition, a mechano-chemical method was used, which comprises the treatment of the mixture of the solid components by intensive mechanical impacts - pressure and shearing deformations, mostly carried out in different kinds of mills which perform impact abrasing actions on the substances. The mixture consisting of the solid betalactam antibiotic substance and finely dispersed nanostructured silica dioxide taken in the ratio of from 10:1 to 75:1 by weight, is exposed to mechanical activation in bead mills. The method used for preparing the mixture helps in a certain way to avoid chemical degradation and to achieve full homogeneity of the powdery components in comparison with making the mixture by a simple mixing of the components or evaporating their solutions, and as a consequence causes a high pharmacological activity of the pharmaceutical composition.

As a quantitative criterion of the minimum necessary dose of mechanical impact it is comfortable to use the granulometry method of the composition suspension. It is necessary that the mass fraction of the particles having a size of not larger than 5 micron is not less than 25%. On the other hand it is necessary to avoid an excessive mechanical treatment which can cause the chemical degradation of betalactams which level can be controlled by known analytical methods, such as HPLC.

The mechanical treatment of the powdery mixtures is performed in rotary, vibrational and planetary mills. Balls, cores and the like can be used as grinding bodies.

Pharmacological tests of the obtained compositions carried out with laboratory animals (mice) showed that the compositions prepared by the method according to the present invention have a higher therapeutic efficiency while treating bacterial sepsis, provoked by *Staphylococcus aureus, Escherichia coli* and *Pseudomonas aeruginosa,* compared with the initial antibiotics.

In such a manner, using the mentioned pharmaceutical compositions and their production process provides the following advantages:
1) Clinically significant increase of the effectiveness and quality of the antimicrobial therapy of semi-acute and acute infection inflammatory diseases, death rate reduction;
2) Ecological safety, lack of wastes and low price of pharmaceutical production technology.
The present invention is illustrated by the following examples.

### Example No. 1. Production of a solid composition of betalactam antibiotic - finely dispersed nanostructured silica dioxide.

The mixtures of the betalactam antibiotic and BHSiO₂ in weight ratios of 10:1, 20:1; 30:1 and 40:1 are processed in an orbicular rotary mill for 1, 2 and 4 hours. The data of the granulometric composition of the aqueous suspension as well as the HPLC analysis of the antibiotic content (in % from the initial substance) are listed in Table No. 2.

**Table No. 2**

| **Granulometric composition of aqueous suspensions and antibiotics content in different composition variations** | | | | |
|---|---|---|---|---|
| Composition content, time m/a* | Dimension and content % of BHSiO₂ particles** | | | Antibiotic content (%) |
| | %<3 micron | %<5 micron | %< 10 micron | |
| Initial BHSiO₂ | 0,5 | 5,3 | 25,7 | - |
| Cefotaxime:BHSiO₂ (10:1), m/a 1 hour | 13,4 | 30,4 | 57,3 | 89 |
| Cefotaxime:BHSiO₂ (20:1), m/a 1 hour | 16,6 | 33,9 | 59,1 | 95 |
| Cefotaxime: BHSiO₂ (40:1), m/a 1 hour | 13,1 | 27,7 | 47,9 | 97 |
| Cefotaxime:BHSiO₂ (30:1), m/a 2 hours | 14,7 | 30,6 | 54,1 | 99 |
| Cefuroxime:BHSiO₂(30:1), m/a 2 hours | 22,6 | 35,2 | 50,2 | 97 |
| Ceftazidime:BHSiO₂ (30:1), m/a 2 hours | 14,3 | 25,3 | 37,0 | 98 |
| Ceftazidime:BHSiO₂ (30:1), m/a 4 hours | 23,8 | 38,9 | 56,2 | 96 |
| Cefepime: BHSiO₂(30:1), m/a 2 hours | 23,8 | 38,8 | 57,7 | 92 |
| Ceftriaxone:BHSiO₂ (30: 1), m/a 1 hour | 24,2 | 43,9 | 66,2 | 97 |
| Ceftriaxone:BHSiO₂ (30:1), m/a 2 hours | 19,4 | 34,5 | 52,4 | 99 |
| Ceftriaxone:BHSiO₂(30:1), m/a 4 hours | 14,5 | 26,4 | 41,7 | 95 |
| Ceftriaxone:BHSiO₂(40:1), m/a 1 hour | 23,4 | 41,2 | 59,1 | 98 |
| Aztreonam:BHSiO₂ (30:1), m/a 2 hours | 21,7 | 39,4 | 53,6 | 97 |
| Meropenem: BHSiO₂ (30: 1), m/a 2 hours | 19,1 | 32,9 | 47,3 | 98 |
| Aztreonam: BHSiO₂ (30:1), m/a 2 hours | 19,8 | 31,1 | 49,5 | 97 |
| Carbenicillin: BHSiO₂(30:1), m/a 2 hours | 22,3 | 38,9 | 51,4 | 96 |

| | | | | |
|---|---|---|---|---|
| * - finely dispersed nanostructured silica dioxide **- mechanical activation | | | | |

As can be seen from Table No. 2, the conditions chosen for the production of the composition allow to increase the part of the finely dispersed BHSiO₂ fraction (particles size less than 5 micron) until a certain value (not less than 25% of the total weight) and to avoid the chemical degradation of the antibiotic.

### Example No. 2. Determination of the therapeutic efficiency of antimicrobial preparations and pharmaceutical compositions.

There has been a research of betalactam antibiotics (Cefazolin, Cefuroxime, Cefotaxime, Ceftriaxone, Cefoperazone, Cefoperazone/sulbactam, Ceftazidime, Cefepime, Cefoxitin, Aztreonam, Meropenem, Carbenicillin) and their compositions mechanized for 2 hours and composed of a mixture of antibiotic / BHSiO₂ in a weight ratio of 30:1, consequently (Cefazolin/ BHSiO₂, Cefuroxime/ BHSiO₂, Cefotaxime/ BHSiO₂, Ceftriaxone/ BHSiO₂, Cefoperazone/ BHSiO₂, Cefoperazone/sulbactam/ BHSiO₂, Ceftazidime/ BHSiO₂, Cefepime/ BHSiO₂, Cefoxitin/ BHSiO₂, Aztreonam/ BHSiO₂, Meropenem/ BHSiO₂, Carbenicillin/ BHSiO₂).

To determine the therapeutic efficiency of betalactams and their pharmaceutical compositions including BHSiO₂, experimental sepsis models and a statistical processing method of the received data (χ²) according to [22, 23] were used.

Microorganisms: *Staphylococcus aureus* (ATCC Nº 25923 F-49), *Escherichia coli* (ATCC Nº25922 F-50), *Pseudomonas aeruginosa* (ATCC Nº27853 F-51).

Animals: for the experiments hybrid mice (CBA x C₅₇Black/₆)CBF₁ according to the "Regulations for test animals use" (USSR Ministry of health order supplement Nº755 from 12.08. 1977) were used.

### Experimental sepsis models:

The mice were injected 0,8ml of a suspension of a daily culture of *Pseudomonas aeruginosa* in a dosage of 5x10⁸ CFU/mouse or a suspension of daily culture of *Staphylococcus aureus* in a dosage of 10¹⁰ CFU/mouse or a suspension of daily culture of *Escherichia coli* in a dosage of 8x10⁸ CFU/mouse. The control group was injected 0,8ml of normal saline solution (0,9% sodium chloride solution). Within one day after being infected, the test mice were daily (during 3 days) intravenously injected 100mg/kg of antibiotics or different pharmaceutical compositions (antibiotic / BHSiO₂) dissolved with 0,25ml of normal saline solution. The control group of mice was injected a normal saline solution of 0,25mg using the same scheme.

The efficiency of the antibacterial therapy was evaluated on the basis of the number of the surviving animals on the 7th day after being infected [22, 23].

The obtained data shown in Table No. 3 reflect the results of 3 independent experiments (for the analysis of each preparation not less than 30 test animals in total were used).

**Table No. 3**

| **Therapeutic efficiency of the antimicrobial therapy of bacterial sepsis** | | | | |
|---|---|---|---|---|
| Tested antibiotics and compositions* | Mice survival rate on the 7^{th} day of infection** | | | χ₂ |
| | *Staphylococcus aureus* | *Escherichia coli* | *Pseudomonas aeruginosa* | |
| Normal saline solution (control) | 0%(0/30) | 0% (0/30) | 0% (0/30) | - |
| Cefazolin | 37,5% (12/32) | - | -*** | P<0,01 |
| Cefazolin/BHSiO₂ | 83,9% (26/31) | - | - | |
| Cefuroxime | 40,0% (14/35) | 43,7% (14/32) | - | P<0,01 |
| Cefuroxime/BHSiO₂ | 84,4% (27/32) | 81,2% (26/32) | - | |
| Cefotaxime | 40,0 % (12/30) | 43,3% (13/30) | - | P<0,01 |
| Cefotaxime/BHSiO₂ | 86,7% (26/30) | 83,3% (25/30) | - | |
| Ceftriaxone | 46,7% (14/30) | 41,9% (13/31) | - | P<0,01 |
| Ceftriaxone/BHSiO₂ | 90,0% (27/30) | 87,5% (28/32) | - | |
| Cefoperazone | - | 45,2% (14/31) | 40,0% (12/30) | P<0,01 |
| Cefoperazone/BHSiO₂ | - | 90,0% (27/30) | 80,6% (25/31) | |
| Ceftazidime | - | 38,7% (15/31) | 43,3% (13/30) | P<0,01 |
| Ceftazidime/BHSiO₂ | - | 84,8% (28/33) | 86,7% (26/30) | |
| Cefepime | 46,7% (14/30) | 43,7% (14/32) | 46,7% (14/30) | P<0,01 |
| Cefepime/BHSiO₂ | 90,0% (27/30) | 85,3% (29/34) | 90,3% (28/31) | |
| Cefoxitin | 35,2% (15/34) | 46,7% (14/30) | - | P<0,01 |
| Cefoxitin/BHSiO₂ | 87,5% (28/32) | 83,3% (25/30) | - | |
| Aztreonam | - | 77,5% (31/40) | 74,4% (32/43) | P<0,01 |
| Aztreonam/BHSiO₂ | - | 95,0% (38/40) | 95,2% (40/42) | |
| Meropenem | 73,3% (22/30) | 78,0% (32/41) | 73,8% (31/42) | P<0,01 |
| Meropenem/BHSiO₂ | 90,6% (29/32) | 95,0% (38/40) | 95,1% (39/41) | |
| Carbenicillin | 46,7% (14/30) | 43,3 % (13/30) | 43,3% (13/30) | P<0,01 |
| Carbenicillin /BHSiO₂ | 83,3% (25/30) | 86,7% (26/30) | 90,0% (27/30) | |
| Cefoperazone/sulbactam | 56,7% (17/30) | 58,1% (18/31) | 59,3% (19/32) | P<0,01 |
| Cefoperazone/sulbactam BHSiO₂ | 86,7% (26/30) | 93,3% (28/30) | 93,5% (29/31) | |

| | | | | |
|---|---|---|---|---|
| *- mixtures having been subjected to mechanical activation for 2 hours and being composed of betalactam antibiotic:finely dispersed nanostructured silica dioxide (BHSiO₂) in a weight ratio of 30:1 **- survival rate/infected animals rate measured in % and absolute values ***- tests were not conducted because of the microorganisms' relatively low-grade sensitivity to initial antibiotics | | | | |

As shown in Table No. 3, all suggested antimicrobially effective pharmaceutical compositions (betalactam/BHSiO₂) definitely possess an increased therapeutic efficiency (1,2 - 2 times higher) compared to simple betalactam in the treatment of sepsis of lab animals, provoked by *Pseudomonas aeruginosa, Staphylococcus aureus* or *Escherichia coli.* These results mostly concern compositions wherein as antibiotics betalactam selected from the group of cefalosporins, cefamicyns and penicillins was used.

### Used literature

1. Antibacterial medicaments. Methods for the standardization of preparations. - M.: JSC «Medicine» Publishing», 2004. - 944 p.
2. M.D. Mashkovsky // Medicaments: Volume 2. - 14th edition. M.: LLC «Novaya Volna Publishing», 2001. - 608 p.
3. Patent RU Nº 2377985 MPK A61K31/43
4. Efficient antibacterial pharmaceutical // Practitioners' Guidance. Under the general editorship of V.P. Yakovlev, S.V. Yakovlev. - M.: Litterra, 2003. -1008 p.
5. A.M. Mayansky // Microbiology for physicians (pathogenetic microbiology essays). - Nizhny Novgorod: Nizhny Novgorod State Medical Academy Publishing, 1999. - 400 p.
6. Abeylath S.C., Turos E. Drug delivery approaches to overcome bacterial resistance to β-lactam antibiotics // Expert Opinion on Drug Delivery. - 2008. - Viol.5. - P.931-949.
7. Bastus N.G., Sanchez-Tillo E., Pujals S. et al. Peptides conjugated to gold nanoparticles induce macrophage activation // Molecular Immunology. - 2009. - Vol.46. - P.743-748.
8. Pinto-Alphandary H., Andremont A., Couvreur P. Targeted delivery of antibiotics using liposomes and nanoparticles: research and applications // International Journal of Antimicrobial Agents. - 2000. - Vol.13. - P.155-168.
9. Ulbrich W., Lamprech A. Targeted drug-delivery approaches by nanoparticulate carriers in the therapy of inflammatory diseases // Journal Royal Society Interface. - 2010. - Vol.7, Suppl. 1. - P.S55-566.
10. A.E. Guliaev, B.A. Ermekbaeva, G.Y. Kivman and etc. Nanoparticles as targeted antibiotic transport (review) // Chemical and pharmaceutical magazine. - 1998. - Nº3. - P.3-6.
11. Rosemary M.J., MacLaren I., Pradeep T. Investigation of antibacterial properties of ciprofloxacin@SiO2. // Langmuir. - 2006. - Vol.22. - P.10125-10129.
12. Rai A., Prabhune A., Perry C.C. Antibiotic mediated synthesis of gold nanoparticles with potent antimicrobial activity and their application in antimicrobial coatings // Journal of Materials Chemistry. - 2010. - Vol.20. - P.6789-6798.
13. Park J-H., Gu L., Maltzahn G. et al. Biodegradable luminescent porous silica nanoparticles for in vivo applications // Nature Materials. - 2009. - Vol.8. - P.331-336.
14. Pernis B. Silica and the immune system // Acta Biomed. - 2005. - Vol.76, Suppl. 2.- P.38-44.
15. Tasciotti E., Liu X., Bhavane R. Et et al. Mesoporous silica particles as a multistage delivery system for imaging and therapeutic applications // Nature Nanotechnology. - 2008. - Vol.3. - P.151-157.
16. Seleem M.N., Munusamy P., Ranjan A et al. Silica-antibiotic hybrid nanoparticles for targeting intracellular pathogens // Antimicrobial Agents and Chemotherapy. - 2009. - Vol.53. - P.4270-4274.
17. Medical chemistry and clinical use of silica dioxide // Edited by NAS of Ukraine academician F.F. Chuyko - Kiev: «Naukova Dumka», 2003. - 416 p.
18. Chuiko A., Pentyuk A., Shtat'ko E., Chuiko N. Medical aspects of application of highly disperse amorphous silica // Surface Chemistry in Biomedical and Environmental Science. Edited by J.P.Blitz and V. Gun'ko.II. Mathematics, Physics and Chemistry. - 2006. - Vol.228. -P.191-204.
19. Lucarelli M., Gatti A.M., Savarino G. et al. Innate defence functions of macrophages can be biased by nano-sized ceramic and metallic particles // European Cytokine Network. - 2004. - Vol.15. - P.339-346.
20. Zolnik B.S., Gonzalez-Fernandez A., Sadrieh N., Dobrovolskaia V. Minireview: Nanoparticles and the immune system // Endocrinology. - 2010. - Vol.151. - P.458-465.
21. N.A. Piataev, A.N. Beliaev, M.D. Romanov, I.S. Kotlov // Pharmacons directed cell assosiated transport. - Saransk: Mordovia University Publishing, 2007. - 140 p.
22. Eckhardt C., Fickweiler K., Schaumann R. et al. Therapeutic efficacy of moxifloxacin in a murine model of severe systemic mixed infection with E.coli and B.fragilis // Anaerobe. - 2003. - Vol.9. - P.157-160.
23. Schaumann R., Blatz R., Beer J. et al. Effect of moxifloxacin versus imipenem/cilastatin treatment on the mortality of mice infected intravenously with different strains of Bacteroides fragilis and Escherichia coli // Journal of Antimicrobial Chemotherapy. - 2004. - Vol.53. - P.318-324.

## Claims

1. An antimicrobial pharmaceutical composition for parenteral administration containing a betalactam antibiotic as therapeutic agent, ***characterized in that*** it is made as a powdery injection preparation and **in that** it contains a betalactam antibiotic and finely dispersed nanostructured silica dioxide in a weight ratio of (10-75):1.

2. The composition according to claim 1, ***characterized in that*** the amount of finely dispersed nanostructured silica dioxide particles having a dimension of not larger than 5 micron is not less than 25%.

3. A process for the preparation of the antimicrobial pharmaceutical composition for parenteral administration, comprising mixing a betalactam antibiotic with other components, ***characterized in that*** the powdery betalactam antibiotic is mixed with powdery finely dispersed nanostructured silica dioxide in a weight ratio of (10-75):1 and the obtained mixture is subjected to mechanical treatment by impact abrasive actions.

4. The process according to claim 3, ***characterized in that*** the obtained mixture is subjected to mechanical treatment by impact abrasive actions, so that the amount of finely dispersed nanostructured silica dioxide particles having a dimension of not larger than 5 micron is not less than 25%.
